# EUROPEAN PATENT APPLICATION

(11) **EP 1 757 571 A1**
(43) Date of publication of application: **28.02.2007**
(21) Application number: 05384030.2
(22) Date of filing: 29.07.2005
(51) Int. Cl.: C07C 29/145, C07C 31/02

(54) **Process for the homogeneous hydrogenation of ketones using ruthenium catalytic systems.**

(71) Applicant: LABORATORIOS DEL DR. ESTEVE, S.A., 08041 Barcelona (ES)
(72) Inventor: Heller, Detleff, 18057 Rostock (DE); Buschmann, Helmut, 08960 Sant Just Desvern (Barcelona) (ES); Drexler, Hans-Joachim, 18198 Gross-Schwass (DE)

(57) **Abstract**

To have an industrial applicable process for the reduction of C₁-C₆ alkyl ketones (including prochiral) or their deuterated derivatives in huge quantities for obtaining their respective alcohols. To provide a new process for the preparation of secondary alcohols substituted with C₁-C₆ alkyl chain or their deuterated derivatives. The process comprises the hydrogenation of ketones (including prochiral) using Rutenium achiral or chiral catalytic systems to render racemic or nonracemic chiral alcohols or deuterated racemic or nonracemic chiral alcohols under low pressure and room temperature.

## Description

The present invention relates to a new process for the preparation of secondary alcohols substituted with C₁-C₆ alkyl chain and/or their deuterated derivatives. The process comprises the hydrogenation of ketones (including prochiral ketones) using achiral or chiral catalytic systems to render the alcohols and/or deuterated alcohols. More particularly, it relates to a new process for the preparation of alcohols of formula (II) 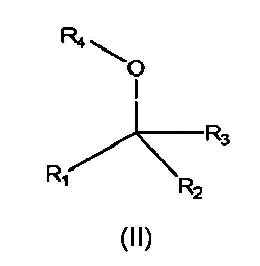 wherein: R₁ and R₂ are independently selected from C₁-C₆ alkyl or deuterated (including partially deuterated) C₁-C₆ alkyl, R₃ and R₄ are independently selected from hydrogen or deuterium in bulk quantities that allows successful technical applications.

The presence of high amounts of C₁-C₆ alkyl substituted ketones as solvents or by-products represents a challenge for the industry because they have to be treated from a recovering point of view internally or through a wastewater plant treatment due to the safety aspects related. Isopropanol for example is a very useful intermediate in organic synthesis as well as a conventionally important solvent.

The reduction of ketones (including prochiral) has been proposed in organic synthesis to obtain secondary alcohols but there is still a need for an industrial applicable homogeneous catalyzed process.

The present invention proposes a process for obtaining a secondary alcohol of formula (II) 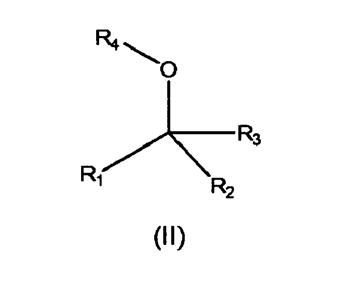 wherein: R₁ and R₂ are independently selected from C₁-C₆ alkyl or deuterated C₁-C₆ alkyl; R₃ and R₄ are independently selected from hydrogen or deuterium; characterized in that a ketone of formula (I) or a prochiral ketone (when R₁ and R₂ are different) 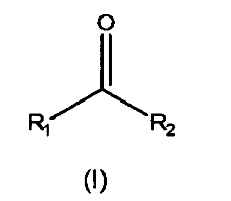 is hydrogenated in the presence of an achiral or chiral ruthenium (II) catalyst system comprising at least a bidentate phosphorous-containing ligand and a diamine ligand at low pressure and room temperature.
When R₁ and R₂ are different an enantiomerically enriched alcohol is obtained using a chiral ruthenium (II) catalyst.

The bidentate phosphorous-containing ligand is a bisphosphine ligand comprising preferably a biaryl group (for example BIPHEP, BINAP).

The diamine is preferably an 1,2 diamine, for example selected from the group consisting of 1,2-ethylendiamine (EN), 1,1-bis(4-methoxyphenyl)-3-methyl-1,2-butanediamine (DAIPEN), 1,2-diphenylethylendiamine (DPEN) or 1,2-diaminacydohexane (DACH).

In the reduction process there are two main decisive factors in the reaction, TON and TOF, defined as follows:
**TON** = [product]/[catalyst]
   When the reduction reaction takes place at low pressure usually the reaction time described is extended proportionally. The catalysts are often not stable enough to result an analogous TON value like is obtained under high pressure conditions.
**TOF** = [product]/([catalyst] time]
   The rate law considering the hydrogen pressure (if investigated) is normally 1^{st} order. Therefore the published TOF's at high pressure can in general be normalized via dividing by the pressure so the literature results given for higher pressure decrease at normal pressure.

For "bulk alcohols" as products especially the efficiency of the catalyst (TON) is of importance. Furthermore a maximal TOF is also needed for production of such bulk products.
In this invention the term "bulk", in respect of alcohols, means alcohols that are not packaged. Also in this invention the term "low pressure" means the range from 0.5 to 20 bar, preferably from 0.8 to 5 bar and more preferably 1 bar.

Finally the term "C₁-C₆ alkyl" means an alkyl group selected from methyl, ethyl, n-propyl, isopropyl, n-butyl, isobutyl, *t*-butyl, n-pentyl and n-hexyl.

In the literature, for example, in the direct hydrogenation of acetone to isopropanol the described Ru-catalysts are only active under high pressure. Furthermore, either the TOF's normalized to 1 bar are too small (33 in maximum) or the temperatures are much higher than room temperature (85°C-150°C) (see for instance FREDIANI, P., et al. J. Organomet. Chem., 1995, vol.498, p.187-197. ; SHVO, Y., et al. J. Organomet. Chem., 1986, vol.315, p.C25-C28. ; GREY, R.A., et al. J. Am. Chem. Soc., 1981, vol.103, p.7536-7542. ; SANCHEZ-DELGADO, R.A., et al. J. Organomet. Chem., 1980, vol.202, p.427-434. or SANCHEZ-DELGADO, R.A., et al. J. Molecular Catal., 1979, p.303-305.).

Published results with other metal compiexes (Rh, Cr, Mo, W, Os) are remarkable worse than the results gained with Ru so that these systems are not relevant for practical application (see for instance DAHLENBURG, L., et al. J. Organomet. Chem., 2000, vol.616, no.19-28. ; MARKO, L., et al. J. Organomet. Chem., 1985, vol.285, p.193-203. ; SANCHEZ-DELGADO, R.A., et al. J. Chem. Soc., Dalton Trans., 1985, p.1859-1863. ; SANCHEZ-DELGADO, R.A., et al. J. Chem. Soc., Chem. Commun., 1983, p.444-445. or FUJITSU, H., et al. J. Chem. Soc., Perkin Trans I. ,1981, p.2650-2655. ).

Furthermore for the direct hydrogenation of 3-pentanone to 3-pentanol there can be found dramatically less catalyst systems than for the corresponding acetone hydrogenation. As already mentioned for acetone Ru is more favourable than other transition metals. Nevertheless, the needed pressure and/or temperature are much to high for a successful technical application (see for instance BLUM, Y., et al. Organometallics., 1995, vol.4, p.1459-1461. ; KIMMRICH, B.F.M., et al. Chem. Commun., 2004, p.1014-1015. ; VOGES, M.H., et al. J. Chem. Soc., Dalton Trans., 2002, p.759-770. or BULLOK, R.M., et al. J. Am. Chem. Soc., 2000, vol. 122, p.12594-12595.). Finally for the direct hydrogenation of 2-butanone to 2-butanol surprisingly no direct hydrogenation with Ru-complexes are given in literature (only transfer hydrogenations and stoichiometric reactions are known.). Until now only achiral Rh-complexes were used which are catalytically active under mild conditions (room temperature and pressure up to 4 bar) but the published TON's and TOF's are too low (see for instance BURK, M.J., et al. Tetrahedron Lett., 1994, vol.35, p.4963-4966. ; TANI, K., et al. Chem. Lett., 1982, p.261-264. or FUJITSU, H., J. Chem. Soc., Perkin Trans I., 1981, p.2650-2655.). There is no information to accessible enantioselectivities.

Surprisingly, the inventors have achieved a very active hydrogenation (high TON's, high TOF's) with achiral and chiral ruthenium (II) catalyst systems of a C₁-C₆ alkyl ketone (induding prochiral) or its deuterated derivatives at low pressure and room temperature.

The object of the present invention was to provide a process for the hydrogenation of C₁-C₆ alkyl ketones (induding prochiral) or their deuterated derivatives with a ruthenium (II) catalyst systems at low pressures and room temperature. This process should operate particularly well on industrial scale and be satisfactory as regards yield, conversion and, wherein is the case, enantiomer excess. In particular the process should be suitable for providing in an advantageous manner specific alcohols and/or enantiomer-enriched alcohols where the efficiency of the catalyst (TON and TOF) is of importance.

A further advantage of the procedure accordingly to the invention is the easy approach to deuterated derivatives of such alcohols which are normally difficult to produce (for instance CH₃-CD(OD)-CH₃; CD₃-CH(OH)-CD₃ achieved by deuteration of acetone or hydrogenation of deuterated acetone respectively).

The different reagents and conditions for the process of the invention will be discussed below.

The ruthenium (II) catalyst system used in the process of the present invention is known to the person skilled in the art and is composed of Ruthenium (II) complexes with two different ligands, a bidentate phosphorous-containing ligand and a diamine, if necessary, in the presence of a base. Said catalyst system components can be provided to the reaction mixture individually to form the reactive catalyst system *in situ* or they can be provided as preformed complexes.

The bidentate phosphorous-containing ligand is in general of the biphosphines or biphosphites types, more preferably it is of the biphosphine type. Illustrative examples of achiral diphosphines are [1,1'-biphenyl]-2,2'-diylbis[diphenylphosphine] (BIPHEP) and derivatives (SCHLOSSER, M. J. Organomet. Chem., 1996, vol.507, p.257-261.), illustrative examples of nonracemic chiral diphosphines are 2,2'-bis(diphenyl-phosphino)-1,1'-binaphtyl (BINAP), TolBINAP and XylBINAP (NOYORI, R., Angew. Chem., Int. Ed. Engl., 2001, vol.40, p.40-73.), 2,2'-bis(diphenylphosphino)-1,1'-dicydopentane (BICP) (CAO, P., et al. J. Org. Chem., 1999, vol.64, p.2127-2129), 2,2',6,6'-tetramethoxy-4,4'bis3,3'-bipyridine (P-Phos), Tol-P-Phos and Xyl-P-Phos (WU, J., et al. J. Org. Chem., 2002, vol.63, p.7908-7910), 4,12-bis(diphenylphos phino)[2.2]paracyclophane (PhanePhos) and Xyl-PhanePhos (BURK, M.J., et al. Org. Lett., 2000, vol.2, p.4173-4176) and equivalents thereto that are recognized by those skilled in the art.

In one preferred embodiment the diphosphine ligand comprises a biaryl group. They are more preferably selected from the group consisting of [1,1'-biphenyl]-2,2'-diylbis[diphenylphosphine] (BIPHEP)(see SCHLOSSER, M. above), 2,2'-bis(diphenyl-phosphino)-1,1'binaphtyl (BINAP), ToIBINAP and XyIBINAP (see NOYORI, R, et al. above).

Suitable diamines are 1,2-diamine species that exhibit a sufficient activity and/or selectivity in the catalyst under consideration. They can be chiral or nonchiral. Illustrative examples are any stereoisomers of 1,1-bis(4-methoxy phenyl)-3-methyl-1,2-butanediamine (DAIPEN), 1,2-diphenylethylendiami ne (DPEN), 1,2-diaminocyclohexane (DACH) or achiral diamines such as ethylenediamine (EN). Achiral amines are further discussed in US 6,743,921 (DSM CATALYTICA PHARMACEUTICALS; 31.07.2003) which is incorporated herein by reference in its entirety.

The bidentate phosphorous-containing ligand together with the diamine and the ruthenium (II) form a complex referred to hereinafter as the ruthenium (II) component of the catalyst system. Examples of preformed complexes of the ruthenium with the diphosphine ligand and the diamine indude complexes represented by the formula RuXYLA wherein X represents a halogen atom, pseudo-halide group or a borhydride group, Y represents a halogen atom, pseudo-halide group or a hydrogen atom. L represents the diphosphine ligand and A is the diamine. Suitable examples are RuCl₂(BIPHEP)(EN), RuCl₂(NNPCy)(EN), RuCl₂(BIPHEP)(Phenylen diamin), RuCl₂(NNPCy)(Phenylendiamin), RuCl₂ (BIPHEP)((R,R)-DPEN), RuCl₂(NNPCy) ((R,R)-DPEN), RuCl₂(BIPHEP)((R)-DAIPEN), RuCl₂(NNP Cy)((R)-DAIPEN) RuCl₂ [(S)-BINAP][(R,R)-DPEN], RuCl₂ [(S)BINAP] [(S,S)-DPEN], RuCl₂[(R)-BINAP] [(R,R)-DPEN], RuCl₂[(R)BINAP] [(S,S)-DPEN], RuCl2 [(R)-BINAP][(R)-DAIPEN], RuCl₂[(S)BINAP][(S)-DAIPEN].

Said component is present in catalytic amounts, meaning less than stoichiometric relative to the ketone reactants and as low as possible while ensuring the optimum possible conversion rate. The minimum amount of the ruthenium (II) component of the catalyst system may depend on the activity of the specific catalyst system composition, the reaction temperature, the concentration of the reactants and catalyst system components in the solution, and the maximum time allowed for completion of the reaction. In a typical embodiments the molar ratio of the ruthenium (II) component of the catalyst to the ketone reactant (s/c) is in the range from about 50 to 2000000, preferably from about 100000.

Suitable bases (if necessary) indude organic bases and inorganic bases which should not have a negative influence on, for example, the enantiomeric purity of the products that are formed if this is the case. Preferably, the base is selected from the group consisting of a hydroxide, C₁-C₅-alkoxide, bicarbonate, carbonate, di- and tribasic phosphate, borate, fluoride, amine optionally substituted with C₁-C₄-alkyl or aryl, or silane optionally substituted with C₁-C₃-alkyl.

In this connection alkali metal alcoholates are advantageous, such as for example *t*-BuOK, as well as inorganic bases such as for example KOH or K₂CO₃. Also organic nitrogen bases such as NEt₃ and salts as for example AgCF₃SO₃ are used. In a more preferred embodiment *t*-BuOK is used. When the base used is *t*-BuOK it is preferably added to the reaction vessel in form of a solution of *t*-BuOK in *t*-BuOH or *i*-PrOH.

It has been found that a molar excess of base referred to the ruthenium (II) component of the catalyst system is advantageous. The typical mole ratio base : ruthenium (II) component of the catalyst system is comprised between 20 000:1 and 1, more preferably between about 100:1 and about 4:1. It has been found that both the activity and (as the case may be) the selectivity of the hydrogenation vary with the amount of the base. Furthermore, if the concentration of base is too high there is a possibility of racemization of the end product, which is not desirable in case of a chiral product.

The hydrogenation reaction is conducted in a solvent system that is capable of dissolving the catalyst system and is reaction-inert. The term solvent system is used to indicate that a single solvent or a mixture of two or more solvents can be used. The term reaction-inert is used to mean that the solvent system does not react unfavourably with the reactants, products, or the catalyst system. The solvent system need not bring about complete solution of the ketone (induding prochiral) reactant or the alcohol or chiral alcohol product. It is preferred to carry out the reaction without additional solvent. Representative solvents are alcohol solvents such as methanol, ethanol, n-propanol, isopropanol, n-butanol, *sec*-butanol or *tert*-butanol and their mixtures, organic solvents containing heteroatoms such as DMF and ethers like THF. Addition of product as solvent can be advantagous. Preferably the solvent system comprises an alcohol solvent, more preferably methanol, isopropanol, *t*-butanol and their mixtures. Isopropanol is particularly preferred.

The hydrogenation takes place in a suitable reactor known to the person skilled in the art, such as an autodave. It is advisable to carry out the hydrogenation under an inert gas atmosphere. Suitable media are nitrogen gas or a noble gas such as argon.

The temperature during the reaction may in principle be chosen arbitrarily by the person skilled in the art as long as a sufficient quick and (as the case may be) selective reaction is guaranteed. However, it has to be taken into account that the temperature depends strongly on solvent and that some catalyst systems are instable above 40 °C. In typical embodiments the reaction is suitably conducted at a temperature comprised from 10 to 60°C, preferably from 20 to 40°C.

The hydrogenation refers to reacting the ketone with a source of hydrogen atoms under appropriate conditions so that two hydrogen atoms are added to the carbonyl group of the ketone to produce the hydroxyl group of the alcohol (including chiral alcohol). Preferably the source of hydrogen atoms includes molecular hydrogen (H₂). If the hydrogenation is carried out in the presence of molecular hydrogen, the hydrogen pressure in the reaction is preferably low, typically at least about a 1.3 bar. Normaly it is in the range from 0.8 to 200 bar. More typically the hydrogen pressure is in the range from 1.3 to 20 bar.

Normally the ketone substrate (I), the catalyst system, (if necessary) the solvent system and (if necessary) the base are introduced in the reactor. The reactor is brought to the adequate temperature and pressure to complete the reaction.

The ketone amount ranges (c/s) from about 1:200000, preferably from about 100000. In general the reaction is allowed to continue until complete conversion of the ketone.

The advantages associated with the invention are numerous: The process according to the invention provides a simple means of access to for instance iso-propanol. The process can be carried out on a large industrial scale with excellent productivity. The process according to the invention makes it possible to prepare the desired product not only in high yields up to complete conversion but also with enantioselectivity using a chiral catalyst and a prochiral substrate. In most cases no additional purification steps are needed, the products may be further processed directly just as they occur.

Accordingly with the invention is also possible to manage huge quantities of bulk ketones in order to recyde it using as solvents that are very useful in the chemical industry. Conversions of 100% of the ketone are achieved by the process of the present invention. Since the constituents of the catalyst (diamine, ruthenium (II) and bidentate phosphorous containg ligand) may be used in several diasteromeric and enantiomeric forms and the complex in each case may therefore be present in so-called matched or mismatched configurations with regard to the chiral ketone, the person skilled in the art must check which pair works most suitably regards selectivity.

The following examples will further illustrate the invention; they should not be interpreted as limiting the scope of the invention.

### General procedure

The whole procedure was carried out under an argon atmosphere.

Reactions under normal pressure

The catalyst system (either a preformed catalyst or a system of Ru-diphosphin catalyst and diamine) was placed in a temperaturable two neck reaction vessel. This is connected to a normal pressure registration equipment. A dropping funnel containing the substrate, (if necessary) the solvent and (if necessary) the base. After adding the solution from the dropping funnel the reaction mixture was stirred and argon was replaced with hydrogen. The reaction was monitored via hydrogen uptake and/or GC-analysis.

### Table 1. Preparation of i-PrOH from acetone

**Table 1**

| **Entry** | **s/c** | **catalyst system** | **substrate base: catalyst** | **added i- PrOH** | **pressure temperature** | **time conversion** | **TON TOF** |
|---|---|---|---|---|---|---|---|
| 1 | 20400 | Ru((S)- | 15 mL | 15 mL | 1 bar | 16 h | 20 400 |
| | | BINAP)Cl₂/ (S,S)- | 50 | | 25°C | 100 % | 1 275 h⁻¹ |
| | | DPEN (in situ) | | | | | |
| 2 | 17 000 | Ru(BIPHEP)(EN) | 12.5 mL | 12.5 mL | 1 bar | 1.4 h | 1700 |
| | | Cl₂ (prepared | 4 | | 25°C | 10 %* | 1 214 h⁻¹ |
| | | catalyst) | | | | | |
| 3 | 20400 | Ru((S)- | 15 mL | 15 ml | 1 bar | 1.2 h | 2856 |
| | | BINAP)((S,S)- | 0 | | 25°C | 14 %* | 2 380 h⁻¹ |
| | | DPEN)(H)HBH₃ | | | | | |

| | | | | | | | |
|---|---|---|---|---|---|---|---|
| * The reaction was not monitored longer. In general, the completely monitored reactions under normal pressure lead to full conversion. | | | | | | | |

### Table 2. Preparation of 2-butanol from 2-butanone

**Table 2**

| **entry** | **s/c** | **catalyst system** | **substrate base: catalyst** | **added i- PrOH** | **pressure temperature** | **time conversion ee** | **TON TOF** |
|---|---|---|---|---|---|---|---|
| 4 | 16 500 | Ru((S)-BINAP)C6/ | 15 mL | 15 mL | 1 bar | 15 h | 16,500 |
| | | (S,S)-DPEN (in | 50 | | 25 °C | 100 % | 1 500 h⁻¹ |
| | | situ) | | | | 15 % (R) | |
| 5 | 11000 | Ru((S)-BINAP)Cl₂/ | 10 mL | 10 mL | 1 bar | 16 h | 11,000 |
| | | (S)-DAIPEN (in | 50 | | 25 °C | 100 % | 1 960 h⁻¹ |
| | | situ) | | | | 21 % (R) | |

### Entry 1

I-PrOH was prepared from 15 mL (340 mmol) acetone, using 0.01 mmol Ru((S)-BINAP)Cl₂ (7.9 mg), 0.01 mmol (S,S)-DPEN (2.1 mg), 15 mL i-PrOH (318 mmol) and 0.5 mmol t-BuOK (1 mL, t-BuOK 0.5 M solution in t-BuOH) at 25°C and 1 bar.
Conversion: 100 % after 16 h, TON = 20 400, TOF=1 275 h⁻¹.

### Entry 2

I-PrOH was prepared from 12.5 mL (170 mmol) acetone, using 0.01 mmol Ru(BIPHEP)(EN)Cl₂ (7.5 mg), 12.5 mL (265 mmol) i-PrOH and 0.04 mmol t-BuOK (0.08 mL, t-BuOK 0.5 M solution in t-BuOH) at 25°C and 1 bar.
Conversion: 10 % after 1.4 h, TON=1 700, TOF=1,214 h⁻¹. Remark: The try was stopped after 1.4 h.

### Entry 3

I-PrOH was prepared from 15 mL (204 mmol) acetone, using 0.01 mmol Ru((S)-BINAP)((S,S)-DPEN)(H)H-BH₃ (9.4 mg) and 15 mL (318 mmol) i-PrOH at 25°C and 1 bar.
Conversion: 14 % after 1.2 h, TON=2 856, TOF=2 380 h⁻¹. Remark: The try was stopped after 1.2 h.

### Entry 4

2-BuOH was prepared from 15 mL (165 mmol) 2-butanone, using 0.01 mmol Ru((S)-BINAP)Cl₂ (7.9 mg), 0.01 mmol (S,S)-DPEN (2.1 mg), 15 mL i-PrOH (318 mmol) and 0.5 mmol t-BuOK (1 mL, t-BuOK 0.5 M solution in t-BuOH) at 25°C and 1 bar.
Conversion: 100 % after 15 h, selectivity: 15 % ee (R), TON=22 500, TOF=1 500 h⁻¹.

### Entry 5

2-BuOH was prepared from 10 mL (110 mmol) 2-butanone, using 0,01 mmol Ru((S)-BINAP)Cl₂ (7.9 mg), 0.01 mmol (S,S)-DAIPEN (2.1 mg), 10 mL i-PrOH (265 mmol) and 0.5 mmol t-BuOK (1 mL, t-BuOK 0.5 M solution in t-BuOH) at 25°C and 1 bar.
Conversion: 100 % after 16 h, selectivity: 21 % ee (R), TON=1 5000, TOF=938 h⁻¹.

## Claims

1. A process for obtaining a secondary alcohol of formula (II) 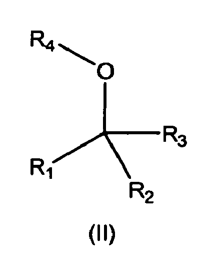 wherein R₁ and R₂ are independently selected from C₁-C₆ alkyl or deuterated C₁-C₆ alkyl and R₃ and R₄ are independently selected from hydrogen and/or deuterium;
**characterized in that** a ketone of formula (I) or a prochiral ketone (when R₁ and R₂ are different) 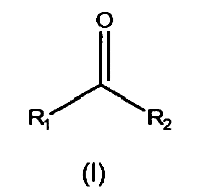 is hydrogenated, in the presence of a base and a achiral or chiral ruthenium (II) catalyst system comprising at least a bidentate phosphorous-containing ligand; a diamine ligand and, if necessary, a base; at low pressure and room temperature.

2. The process according to daim 1 **characterized in that** when R₁ and R₂ are different an enantiomer enriched alcohol is obtained.

3. The process according to daim 1 and 2, wherein the bidentate phosphorous-containing ligand is a bisphosphine ligand comprising a biaryl group, preferably selected from the group formed by 1,1'-biphenyl-2,2'-diylbis(diphenyl phosphine) (BIPHEP), stereoisomers of 2,2'-bis(diphenyl-phosphino)-1,1'-binaphtyl (BINAP), TolBINAP and XylBINAP.

4. The process according to daims 1 to 3 wherein the diamine is an (if necessary enantiomerically-enriched) 1,2 diamine, preferably selected from the group consisting of 1,2-ethylenediamine (EN), 1,1-bis(4-methoxyphenyl)-3-methyl-1,2-butanediamine (DAIPEN), 1,2-diphenylethylendiamine (DPEN).

5. The process according to daims 1 to 4 wherein the base is selected from alkali metal alcoxide, preferably *t*-butoxide.

6. The process according to daims 1 to 5 wherein the base is selected from *t*-BuOK, K₂CO₃, NEt₃ and AgCF₃SO₃, preferably *t*-BuOK.

7. The process according to daims 1 to 6 wherein the hydrogenation is carried out pressure from 0.5 to 20 bars, preferable from 0.8 to 5 bar and more preferably the process is carried out at 1 bar of pressure.

8. The process according to daims 1 to 7 wherein the solvent is an alcohol, preferably selected from methanol, isopropanol, t-butanol and their mixtures, more preferably is isopropanol.

9. The process according to daims 1 to 8 which further, when R₁ and/or R₂ are independently deuterated C₁-C₆ alkyl and/or R₃ and/or R₄ are independently deuterium comprises a deuteration reaction of the compound of formula II.

10. The process according to daim 9 wherein the deuteration is carried out on the product of the process as defined in daims 1 to 8, without an intermediate separation or purification step.

11. The process according to daims 1 to 10 wherein either of R₁ or R₂ is a C₁-C₆ alkyl selected from: methyl, ethyl, n-propyl, isopropyl, n-butyl, isobutyl, *t*-butyl, n-pentyl and n-hexyl, preferable both are methyl, ethyl, or n-propyl and more preferable both are methyl.
